# EUROPEAN PATENT SPECIFICATION

(11) **EP 0 914 387 B1**
(45) Date of publication and mention of the grant of the patent: **18.06.2003**
(21) Application number: 98912442.5
(22) Date of filing: 05.03.1998
(51) Int. Cl.: C09C 1/00, C09D 7/12, C09D 11/00, C08K 3/00, A61K 7/00

(54) **HIGH-CHROMA ORANGE PEARL PIGMENT**
HOCHCHROMATISCHES ORANGE PERLGLANZPIGMENT
CONCEPTION D'UNE NACRE ARTIFICIELLE ORANGE A FORTE SATURATION DES COULEURS REMPLA ANT UN PIGMENT AU CADMIUM

(30) Priority: 19.03.1997 JP 8471297
(43) Date of publication of application: 12.05.1999
(73) Proprietor: MERCK PATENT GmbH, 64293 Darmstadt (DE)
(72) Inventor: NOGUCHI, Tamio, Merck Japan KK, Onahama koujou nai, Iwaki-shi, Fukushima-ken (JP)
(86) International application number: EP9801243
(87) International publication number: WO98041584

(56) References cited:
- EP-A- 0 077 959
- EP-A- 0 446 689
- EP-A- 0 796 612

## Description

### [Field of the Invention]

This invention relates to a novel orange pearl pigment including fine spherical metal oxide particles containing iron oxide coated on the surfaces of a flaky substrate, which pigment is useful as a coloring material not only in the fields of industrial products, such as paints, inks and plastics, but also in other fields, such as cosmetics.

### [Background Art]

A cadmium pigment has been the only known orange colored pigment. The cadmium pigment is the pigment of a solid solution consisting of cadmium sulfide (CdS) as main component , and appropriate proportions of zinc sulfide (ZnS), cadmium selenide and mercury sulfide (HgS). The cadmium pigment has been widely used, since it has been considered as a coloring agent of high clearness for which there is virtually no substitute (Tsunashima, et al.: Latest Applied Pigment Technology, page 24, C.M.C. Co., Ltd.). The use of the cadmium pigment has, however, been discontinued almost completely, since it bagan to be avoided when the cadmium pollution in environment became a social problem.

On the other hand there have been sold, or proposed pigments which comprise a flaky substrate coated with iron oxide, or a metal oxide containing iron oxide, as described below, but no pearl pigment having an orange color has been developed as yet.

The inventor of this invention previously disclosed a transparent color pigment which comprises mica particles coated thereon with iron oxide and/or a hydrate thereof (see Japanese Patent Publication No. Hei 1-60511). This pigment is, however, an orange pigment having a high degree of transparency and a low hiding power, since its observation by a scanning electron microscope reveals that iron oxide is composed of needle crystals having a diameter of as large as 0.1 to 0.2 micron, and causing, therefore, large scattering of reflected light.

The pearl pigments which comprise mica, or like particles coated with iron oxide are made and sold for practical use by Merck as a series of products under the tradename "IRIODIN 500". They are, however, not pearl pigments having an orange color of high chroma. In connection with two series of pigments comprising mica as a flaky substrate with two coated system of titanium oxide and ferric oxide, respectively, as a coating metal oxide, Thurn-Muller, et al. report the values "a" (indicating red on the + side and green on the - side), and "b" (yellow on the + side and blue on the - side) of the Hunter color tone produced by each such pigment, and by varying with the amount of the coating (or its optical thickness) (Kontakte, No. 2, pages 35-43, 1992). It is understood that the most desirable orange color having the highest chroma is obtained when the maximum values of "a" and "b" coincide with each other. According to this literature, the pigments which comprise mica coated with titanium oxide show only interference colors, but as is obvious from the variations of the values "a" and "b", there is no coincidence between the maximum values of "a" and "b" with respect to any interference color, and there does not exist any range in which an interference orange color of high chroma is produced. Studies have also been made of the pigments which comprise mica coated with ferric oxide, and include the measuring result of variations of the each "a" and "b" value of combined interference color with absorption characteristic of ferric oxide (complementary colors to absorption colors). But the colors vary from bronze to copper, and from copper to sienna, and they fail to show any coincidence between the maximum values of "a" and "b", as the pigments in the system of containing titanium oxide.

There has also been proposed a red pigment having an orange to bluish red color which comprises sheet-like iron oxide particles, or sheet-like particles coated with iron oxide and an aluminum compound layer, or an aluminum compound layer containing a composite of iron oxide and alumina, being coated with such a layer having an appropriate optical thickness
(see Japanese Patent Laid open No. Hei 6-100794). This pigment has reddish color generated by double layer structure such as combination of reflection (complementary) by the absorption of the iron oxide coated on the sheet-like particles, with interference color due to a second coating structure controlling the thickness of the outer aluminum oxide layer. The combined colors of interference and reflection are claimed to give a reddish color of high chroma having a by far sharper tone than that of the color (of reflection) obtained by a pigment containing only iron oxide. The pigment is also claimed to be able to develop a color of still improved chroma if the outer aluminum oxide layer is replaced by a composite oxide layer of iron and aluminum oxides.

EP 0 796 612 discloses a cosmetic composition comprising a filler, a fatty binder and at least one nanopigment, said nanopigment comprising of a metal oxide.

EP 0 446 689 describes platelet-shaped substrates, coated with a metal oxide and at least 10 % per weight of aluminium oxide, utilised in enamels, glazes and paints.

These pigments are, however, produced by a known and commonly used process, i.e. neutralization decomposition, the urea process (uniform precipitation reaction), or thermal hydrolysis, employing iron and aluminum salts as materials for the coating metal compounds, and no pearl pigment having an orange color can be obtained.
Thus, only the cadmium pigment is known as an orange pigment, and there is a demand for the development of a safe and high-chroma orange colored pigment instead of it.

### [Disclosure of the Invention]

As a result of the earnest study for developing an orange pearl pigment under these circumstances, the inventor of this invention has succeeded in developing a novel orange pearl pigment of high luster and chroma by coating the surfaces of a flaky substrate with fine spherical metal oxide particles consisting mainly of spherical particles of iron oxide having a size suited for producing a yellowish red color.

Thus, this invention provides a novel orange pearl pigment, a process for manufacturing the same and use thereof as set forth at 1) to 6) below.
1) An orange pearl pigment comprising a metal oxide containing iron oxide, coated on a flaky substrate, said metal oxide being fine spherical particles comprising said iron oxide in the amount of 40 to 300 parts by weight in terms of ferric oxide relative to 100 parts by weight of said flaky substrate, obtainable by a process in which a sulfate and/or persulfate and/or polysulfate are added to a suspension of the flaky substrate prior to addition of iron salt.
2) The orange pearl pigment as set forth at 1), wherein ammonium sulfate, potassium sulfate, sodium sulfate, potassium aluminium sulfate, ammonium persulfate or sodium pyrosulfate are added to a suspension of the flaky substrate prior to addition of iron salt.
3) An orange pearl pigment comprising a metal oxide containing iron oxide, coated on a flaky substrate, said metal oxide being fine spherical particles comprising said iron oxide in an amount of 40 to 300 parts by weight in terms of ferric oxide relative to 100 parts by weight of said flaky substrate, not more than 35 % by weight of aluminium oxides in terms of Al₂O₃, and not more than 2 % by weight of calcium oxides in terms of CaO, and/or not more than 2 % by weight of magnesium oxides in terms of MgO, relative to said iron oxide in terms of ferric oxide.
4) A process for manufacturing an orange pearl pigment, as set forth at 1), comprising preparing an aqueous suspension of a flaky substrate, adding a sulfate and/or a persulfate and/or a polysulfate into said suspension, heating said suspension under stirring, adding a) an aqueous solution of a ferric salt, and b) an aqueous alkali solution into said suspension, while maintaining said suspension at a pH of 2 to 5, then adding aqueous alKali solution into said suspension until reaching a pH of 8 to 10, separating a product by filtration, washing it, drying it, and calcining it at a temperature not lower than 500°C.
5) A process for manufacturing an orange pearl pigment as set forth at 3), comprising adding into an aqueous suspension of a flaky substrate and heating said suspension under stirring, a) an aqueous solution of a ferric salt and a magnesium salt and/or a calcium salt, and b) an aqueous alkali solution into said suspension, while maintaining it at a pH of 2 to 5, adding additional aqueous alkali solution into said suspension until achieving a pH of 8 to 10, separating a product by filtration, washing it, drying it, and calcining it at a temperature not lower than 500°C.
6) A paint, ink, plastic, or cosmetic containing an orange pearl pigment as set forth at any of 1) to 3) above.

This invention is, for increasing chroma and pearl luster of orange color at visual observation, based on the discovery of the fact that an orange pearl pigment can be obtained by, the size, or crystal form of metal oxide particles containing iron oxide coated on the surfaces of a flaky substrate is controlled, and by, the coating weight thereof is so controlled as to give an optical thickness suited to within the range in which a reddish color of interference is produced (the range between the maximum values of "a" and "b").

The following is a detailed description of the invention.

The flaky substrate which is used for the purpose of this invention is a transparent one, such as mica, synthetic mica, glass flakes or flaky silica, having a particle diameter of 1 to 150 microns ana a tnickness not exceeding 5 microns, and preferably an average thickness not exceeding 1 micron.

The following is a process for manufacturing an orange pearl pigment of high luster and chroma according to this invention. First of all, a flaky substrate is suspended in water, and its suspension is heated to 60°C or above. It is heated to a temperature preferably, say, between 70°C and its boiling point. Although an aqueous solution of an iron salt as will be described later may be added to the suspension to make a pearl pigment coated with iron oxide, it is preferable to add a sulfate and/or a persulfate and/or a polysulfate (hereinafter referred to simply as "sulfate groups"). Any water-soluble salt can be used as the sulfate groups, for example, ammonium sulfate ((NH₄)₂SO₄), potassium sulfate (K₂SO₄), sodium sulfate (Na₂SO₄) and potassium aluminum sulfate (potassium alum: AlK(SO₄)₂) mentioned as the sulfates, ammonium persulfate ((NH₄)S₂O₈), potassium persulfate (K₂S₂O₈) and sodium persulfate (Na₂S₂O₈) as the persulfates, and potassium pyrosulfate (K₂S₂O₇) and sodium pyrosulfate (Na₂S₂O₇) as the polysulfates. It is important to add the sulfate groups before dropping an iron salt, etc. Although the mechanism of action of the sulfate groups is not clear, it is assumed that the addition thereof increases the ionic strength in the suspension, and that they and the large anions of the sulfate groups take part in the formation step of particles of a hydrated metal oxide during the later process of hydrolysis of an additing metal salt such as iron salt, and contribute to controlling their size. It is used in the amount of 0.005 to 0.1 mol relative to 1 mol of an iron salt which will be dropped later, though other conditions may also have to be considered. The use of less than 0.005 mol is not effective for any satisfactory improvement in the chroma of an orange color as intended. In other words, it is impossible to form hydrated metal oxide particles as a precursor for fine spherical metal oxide particles having a diameter suited for the producing an orange color. The use of more than 0.1 mol is not expected to produce any better result, but is undesirable from the standpoint of efficiency of manufacture, since any excess prolongs the later step of washing for removing any free salt. The smaller in size the flaky substrate, the larger specific surface area it has, and it is, therefore, preferable to use as large an amount of the sulfate groups as possible if it is within the above range.

Then, (a) an aqueous solution of a ferric salt and (b) an aqueous alkali solution which are separately prepared are dropped in appropriate amounts into the suspension, while it is maintained at a pH of 2 to 5. It is possible to obtain a pearl pigment having still better properties if (a) the aqueous solution of a ferric salt is replaced by (c) a mixed aqueous solution prepared by adding one or more metal salts selected from among aluminum, magnesium and calcium salts to an aqueous solution of a ferric salt. An aqueous solution of e.g. sodium or potassium hydroxide, or ammonia is used as (b) the aqueous alkali solution. After their dropping, (b) the aqueous alkali solution is added again to the suspension until a pH of 8 to 10, and a dry calcined obtained by successive filtration, washing and drying is calcined at a temperature of 500 °C or above to produce a pearl pigment as intended.

As to the ferric salt, it is possible to use any soluble ferric salt, and it is appropriate to use, for example, a chloride, sulfate, or nitrate. It is preferably used in such an amount that the amount of iron oxide in the pigment may be 40 to 300 parts by weight in terms of ferric oxide relative to 100 parts by weight of flaky substrate. Any variation within the range is possible, and depends on the color tone and properties of the flaky substrate. The use of any amount less than 40% by weight is undesirable, since it fails to form any coating thickness capable of producing any interference color, and since it is greatly, directly, affected by the proper color of the flaky substrate because of low hiding power of coated layer. Any amount over 300% by weight is also undesirable, since there is only produced a interference color which deviates from the desired reddish range. In case of the flaky substrate has a small particle diameter, it is necessary to increase the amount of the ferric salt, since the substrate has a large specific surface area.

It is possible to make an orange pearl pigment of still higher chroma by employing instead of the ferric salt (c) a mixed aqueous solution prepared from a ferric salt and an aluminum and/or calcium and/or magnesium salt. This is assumed due to the fact that the combination of iron and another metal facilitates the sintering of metal oxide particles containing iron oxide, and the densification of the individual fine particles, resulting in a higher apparent refractive index and thereby a higher reflectivity.

The appropriate amounts in which those metal salts are used for the purpose of this invention are now more than 35% by weight of aluminum oxides in terms of Al₂O₃, not more than 2% by weight of calcium oxides in terms of CaO and not more than 2% by weight of magnesium oxides in terms of MgO relative to iron oxide (in terms of ferric oxide). Any excess over these ranges is undesirable, since it adversely affects the hue rather than being effective for the sintering of the individual fine particles. The aluminum, magnesium or calcium oxide, or a composite oxide thereof is also effective for making a pigment powder of improved dispersibility.

As the aluminum, magnesium or calcium salt for the purpose of this invention, it is possible to use any soluble salt of the relevant metal, and it is possible to mention, for example, a soluble metal chloride, sulfate, nitrate, carbonate or acetate.

The manufacturing process of this invention employs a calcining temperature of 500°C or above which enables the formation of fine spherical metal oxide particles having a diameter of 0.02 to 0.1 micron suited for producing a yellowish red color, and the preferred crystal form, while maintaining the size of hydrated metal oxide particles. The preferred range of the calcining temperature is from 600°C to 900°C. If the calcining temperature is too low, there are obtained iron oxide particles containing a large proportion of goethite crystals and having a strong yellowish tinge. If the calcining temperature is high, there are obtained iron oxide particles containing a large proportion of hematite crystals and having a strong reddish tinge. If the calcining temperature is too high, the fine spherical metal oxide particles melt together and form particles having an undesirably large diameter, while also causing the cohesion of particles of the flaky substrate, resulting in a powder which is unsatisfactory in dispersibility. Although the crystal forms of the fine spherical metal oxide particles produced in accordance with this invention are not definitely clear, it is assumed that the crystals exist as a mixture of from goethites to hematites which comes from iron oxide, and also include mixtured aluminum, calcium or magnesium oxide, or a composite oxide in case of that the pigment is made by employing a salt of any such metal, too. Therefore, the calcining temperature and the amount of heat to be applied are so selected as to carry out a process of dehydration crystallization which enables the fine spherical hydrated metal oxide particles to retain their size until after drying and calcining and form the crystal form desired for producing of an orange color.

The orange pearl pigment manufactured as described above has a high level of chroma, since as & result of its colorimetrie measurement by CIE (values L, a and b), it shows a value L of 55 to 70, a value "a" of 20 to 40 and a value "b" of 35 to 55 in the white background, and a value L of 50 to 65, a value "a" of 10 to 30 and a value "b" of 20 to 35 in the black background. The pigment can be used in paints, plastics, inks or cosmetics. The invention will now be described in further detail by way of examples, though these examples are not intended for limiting this invention.

### [Examples]

### Example 1

A suspension was prepared by adding 119 g of mica powder having a particle diameter of 10 to 60 microns to 1.5 liters of water, and after the addition of 4.0 g of potassium sulfate, the suspension was heated to 85°C under stirring. An aqueous solution prepared by dissolving 154.7 g of ferric chloride in 0.83 liter of water was dropped into the suspension, while an aqueous alkali solution was used to maintain it at a pH of about 3.0. The aqueous alkali solution was thereafter added again to the suspension until a pH of 8.5. Then, a solid product was separated from the suspension by filtration, washed, dried, and calcined at about 880°C to produce an orange pearl pigment as shown in Table 1.

### Example 2

A suspension was prepared by adding 119 g of mica powder having a particle diameter of 10 to 60 microns to 1.5 liters of water, and after the addition of 2.0 g of potassium persulfate, the suspension was heated to 85°C under stirring. An aqueous solution prepared by dissolving 154.7 g of ferric chloride in 0.83 liter of water was dropped into the suspension, while an aqueous alkali solution was used to maintain it at a pH of about 3.0. The aqueous alkali solution was thereafter added again to the suspension until a pH of 8.5. Then, a solid product was separated from the suspension by filtration, washed, dried, and calcined at about 880°C to produce an orange pearl pigment as shown in Table 1.

### Example 3

A suspension was prepared by adding 119 g of mica powder having a particle diameter of 10 to 60 microns to 1.16 liters of water, and after the addition of 3.9 g of potassium persulfate, the suspension was heated to 85°C under stirring. A mixed aqueous solution prepared by dissolving 1 g of magnesium chloride, 1 g of calcium chloride, 19 g of aluminum chloride and 154.7 g of ferric chloride in 0.83 liter of water was dropped into the suspension, while an aqueous alkali solution was used to maintain it at a pH of about 3.0. The aqueous alkali solution was thereafter added again to the suspension until a pH of 8.5. Then, a solid product was separated from the suspension by filtration, washed, dried, and calcined at about 850°C to produce an orange pearl pigment as shown in Table 1.

### Example 4

An orange pearl pigment as shown in Table 1 was made by using potassium pyrosulfate instead of potassium persulfate and otherwise repeating Example 3.

### [Comparative Example]

### Comparative Example 1

A low-chroma reddish orange pearl pigment as shown in Table 1 was obtained by repeating Example 2 without using potassium persulfate.

### [Table 1]

### Table 1 - Amounts of materials used and color tones of pigments obtained (white background)

Example 1 to 4; Comparative Example 1

Note 1): In Table 1, the amount of each metal salt is shown in grams relative to 100 g of mica.
Note 2): #500 is a pearl pigment comprising mica having a particle diameter of 10 to 60 microns and coated with about 61% by weiqht of iron oxide in terms of ferric oxide (Iriodin 500, product of Merck).

Method for measurement of color tones (values L, a and b):
A sample was prepared by mixing 1 part of pigment and 9 parts of PVC (having a solid content of 20%), and applied to black and white hiding paper by a bar coater 20, and after drying, its values L, a and b were determined by a color meter CR-200 (product of Minolta). The values a and b were used to calculate the values of C and ∠H° in accordance with equations: C (chroma) = (a²+b²)^{1/2} and ∠H° (hue angle) = tan⁻¹(b/a). The results are shown in Table 1.

The orange pearl pigments of this invention produce an orange color having a higher level of chroma than those of the conventional pearl pigments, as shown in Table 1, and can be used in the field of paints for automobiles, or for general industrial purposes, in the field of plastics, as not only for the mere color decoration, but also for cases involving plateout, in the field of laser marking, in the field of inks and in the field of cosmetics to produce a vivid orange color having pearl luster.

### [Examples of application]

### Examples of application

The following is a description of examples in which the pearl pigments which had been obtained in the Examples above were used in paints, plastics, inks and cosmetics.

### (1) Example of use in a paint:

This is an example of use in a top coat paint for automobiles.

### {Base paint composition}

### <Acrylic-melamine resin>

| | |
|---|---|
| Acrydic 47-712 (product of Dainippon Ink Co., Ltd.) | 70 parts by weight |
| Super Beccamine G812-60 (product fo Dainippon Ink Co., Ltd.) | 30 parts by weight |
| Toluene | 30 parts by weight |
| Ethyl acetate | 50 parts by weight |
| N-butanol | 10 parts by weight |
| Solvesso #150 (Tonen Chemical) | 40 parts by weight |

A paint was prepared by mixing 100 parts by weight of the above acrylic-melamine resin composition and 20 parts by weight of each of the orange pearl pigments as obtained in Examples 1 to 4, and adding a thinner for the acrylic-melamine resin to the mixture to lower its viscosity to a level suitable for spray coating (12 to 15 seconds with Ford Cup No. #4), and was applied by spray coating to form a base coat layer. An uncolored top clear paint of the following composition was applied onto the base coat layer:

### {Top clear paint}

| | |
|---|---|
| Acrydic 44-179 | 14 parts by weight |
| Superbeccamine L117-60 | 6 parts by weight |
| Toluene | 4 parts by weight |
| Butyl cellosolve | 3 parts by weight |

After top coating, the paints were exposed to the air at 40°C for 30 minutes, and heated for curing at 135°c for 30 minutes.

### (2) Example of use in a plastic:

The following is an example of composition in which the pigments were used for coloring a plastic:

| | |
|---|---|
| Polyethylene resin (pellets) | 100 parts by weight |

Each of the orange pearl pigments as obtained in

| | |
|---|---|
| Examples 1 to 4 | 1 part by weight |
| Zinc stearate | 0.2 part by weight |
| Liquid paraffin | 0.1 part by weight |

The pellets containing the above composition were dry blended, and extrusion molded.

### (3) Example of use in a printing ink:

The following is an example of an ink composition for gravure printing:

| | |
|---|---|
| CCST medium (nitrocellulose resin of Toyo Ink Co., Ltd.) | 10 parts by weight |
| Each of the orange pearl pigments as obtained in Examplse 1 to 4 | 8 parts by weight |

A solvent NC102 (Toyo Ink Co., Ltd.) was added to the ink of the above composition to adjust its viscosity to a level of 20 seconds as measured by Zahn Cup No. 3, and it was used for printing.

### (4) Example for use in a cosmetic:

The following is a composition for a lip-coloring cosmetic:

| | |
|---|---|
| Ozokerite | 5 parts by weight |
| Ceresine | 5 parts by weight |
| Paraffin wax | 10 parts by weight |
| Glycerol trioctanate | 20 parts by weight |
| Diisostearyl malate | 42 parts by weight |
| Octyldodecyl myristate | 10 parts by weight |

Each of the orange pearl pigments as obtained in Examples 1 to 4, and coloring matter: Appropriate amounts Oxidation inhibitor, preservative, and perfume: Small amounts

A lipstick was formed from the above composition.

## Claims

1. An orange pearl pigment comprising a metal oxide containing iron oxide, coated on a flaky substrate, said metal oxide being fine spherical particles comprising said iron oxide in the amount of 40 to 300 parts by weight in terms of ferric oxide relative to 100 parts by weight of said flaky substrate, obtainable by a process in which a sulfate and/or persulfate and/or polysulfate are added to a suspension of the flaky substrate prior to addition of iron salt.

2. The orange pearl pigment according to claim 1, wherein ammonium sulfate, potassium sulfate, sodium sulfate, potassium aluminium sulfate, ammonium persulfate or sodium pyrosulfate are added to a suspension of the flaky substrate prior to addition of iron salt.

3. An orange pearl pigment comprising a metal oxide containing iron oxide, coated on a flaky substrate, said metal oxide being fine spherical particles comprising said iron oxide in an amount of 40 to 300 parts by weight in terms of ferric oxide relative to 100 parts by weight of said flaky substrate, not more than 35 % by weight of aluminium oxides in terms of Al₂O₃, and not more than 2 % by weight of calcium oxides in terms of CaO, and/or not more than 2 % by weight of magnesium oxides in terms of MgO, relative to said iron oxide in terms of ferric oxide.

4. A process for manufacturing an orange pearl pigment, according to claim 1, comprising preparing an aqueous suspension of a flaky substrate, adding a sulfate and/or a persulfate and/or a polysulfate into said suspension, heating said suspension under stirring, adding a) an aqueous solution of a ferric salt, and b) an aqueous alkali solution into said suspension, while maintaining said suspension at a pH of 2 to 5, then adding aqueous alkali solution into said suspension until reaching a pH of 8 to 10, separating a product by filtration, washing it, drying it, and calcining it at a temperature not lower than 500°C.

5. A process for manufacturing an orange pearl pigment according to Claim 3, comprising adding into an aqueous suspension of a flaky substrate and heating said suspension under stirring, a) an aqueous solution of a ferric salt and a magnesium salt and/or a calcium salt, and b) an aqueous alkali solution into said suspension, while maintaining it at a pH of 2 to 5, adding additional aqueous alkali solution into said suspension until achieving a pH of 8 to 10, separating a product by filtration, washing it, drying it, and calcining it at a temperature not lower than 500°C.

6. A paint, ink, plastic, or cosmetic containing an orange pearl pigment according to any of claims1 to 3.

## Patentansprüche

1. Orangefarbenes Perlglanzpigment aus einem auf einem plättchenförmigen Substrat aufgetragenen eisenoxidhaltigen Metalloxid, wobei das Metalloxid als feine kugelförmige Teilchen vorliegt und Eisenoxid in einer Menge von 40 bis 300 Gewichtsteilen, gerechnet als Fe₂O₃ und bezogen auf 100 Gewichtsteile des plättchenförmigen Substrats, enthält, erhältlich indem man eine Suspension des plättchenförmigen Substrats vor Zusatz von Eisensalz mit einem Sulfat und/oder Persulfat und/oder Polysulfat versetzt.

2. Orangefarbenes Perlglanzpigment nach Anspruch 1, bei dem man eine Suspension des plättchenförmigen Substrats vor Zusatz von Eisensalz mit Ammoniumsulfat, Kaliumsulfat, Natriumsulfat, Kaliumaluminiumsulfat, Ammoniumpersulfat oder Natriumpyrosulfat versetzt.

3. Orangefarbenes Perlglanzpigment aus einem auf einem plättchenförmigen Substrat aufgetragenen eisenoxidhaltigen Metalloxid, wobei das Metalloxid als feine kugelförmige Teilchen vorliegt und Eisenoxid in einer Menge von 40 bis 300 Gewichtsteilen, gerechnet als Fe₂O₃ und bezogen auf 100 Gewichtsteile des plättchenförmigen Substrats, höchstens 35 Gew.-% Aluminiumoxide, gerechnet als Al₂O₃, und höchstens 2 Gew.-% Calciumoxide, gerechnet als CaO, und/oder höchstens 2 Gew.-% Magnesiumoxide, gerechnet als MgO, bezogen auf das Eisenoxid, gerechnet als Fe₂O₃, enthält.

4. Verfahren zur Herstellung eines orangefarbenen Perlglanzpigments gemäß Anspruch 1, bei dem man ein plättchenförmiges Substrat in eine wäßrige Suspension überführt, mit einem Sulfat und/oder einem Persulfat und/oder einem Polysulfat versetzt, unter Rühren erhitzt, bei einem konstant gehaltenen pH-Wert von 2 bis 5 mit a) einer wäßrigen Lösung eines Eisen(III)-salzes und b) einer wäßrig-alkalischen Lösung versetzt, anschließend mit wäßrig-alkalischer Lösung auf einen pH-Wert von 8 bis 10 einstellt, abfiltriert, wäscht, trocknet und bei mindestens 500°C kalziniert.

5. Verfahren zur Herstellung eines orangefarbenen Perlglanzpigments gemäß Anspruch 3, bei dem man eine wäßrige Suspension eines plättchenförmigen Substrats unter Rühren erhitzt, bei einem konstant gehaltenen pH-Wert von 2 bis 5 mit a) einer wäßrigen Lösung aus einem Eisen(III)-salz und einem Magnesiumsalz und/oder einem Calciumsalz und b) einer wäßrig-alkalischen Lösung versetzt, mit weiterer wäßrig-alkalischer Lösung auf einen pH-Wert von 8 bis 10 einstellt, abfiltriert, wäscht, trocknet und bei mindestens 500°C kalziniert.

6. Farbe, Kunststoff oder Kosmetik, enthaltend ein orangefarbenes Perlglanzpigment gemäß einem der Ansprüche 1 bis 3.

## Revendications

1. Pigment nacré orangé comprenant un oxyde métallique contenant de l'oxyde de fer, appliqué sur un substrat lamellaire, ledit oxyde métallique étant constitué de particules sphériques fines comprenant ledit oxyde de fer en une quantité de 40 à 300 parties en poids, exprimée en oxyde ferrique, pour 100 parties en poids dudit substrat lamellaire, pouvant être obtenu par un procédé dans lequel un sulfate et/ou un persulfate et/ou un polysulfate sont ajoutés à une suspension du substrat lamellaire avant addition du sel de fer.

2. Pigment nacré orangé selon la revendication 1, dans lequel du sulfate d'ammonium, du sulfate de potassium, du sulfate de sodium, du sulfate de potassium et d'aluminium, du persulfate d'ammonium ou du pyrosulfate de sodium sont ajoutés à une suspension du substrat lamellaire avant addition du sel de fer.

3. Pigment nacré orangé comprenant un oxyde métallique contenant de l'oxyde de fer, appliqué sur un substrat lamellaire, ledit oxyde métallique étant constitué de particules sphériques fines comprenant ledit oxyde de fer en une quantité de 40 à 300 parties en poids, exprimée en oxyde ferrique, pour 100 parties en poids dudit substrat lamellaire, pas plus de 35 % en poids d'oxydes d'aluminium exprimés en Al₂O₃, et pas plus de 2 % en poids d'oxydes de calcium, exprimés en CaO, et/ou pas plus de 2 % en poids d'oxydes de magnésium, exprimés en MgO, par rapport audit oxyde de fer, exprimé en oxyde ferrique.

4. Procédé de fabrication d'un pigment nacré orangé selon la revendication 1, qui comprend la préparation d'une suspension aqueuse d'un substrat lamellaire, l'addition d'un sulfate et/ou d'un persulfate et/ou d'un polysulfate à ladite suspension, le chauffage de ladite suspension sous agitation, l'addition a) d'une solution aqueuse d'un sel ferrique et b) d'une solution aqueuse alcaline à ladite suspension, tout en maintenant ladite suspension à pH 2 à 5, puis l'addition d'une solution aqueuse alcaline dans ladite suspension jusqu'à atteindre un pH de 8 à 10, la séparation d'un produit par filtration, son lavage, son séchage et sa calcination à une température non inférieure à 500°C.

5. Procédé de fabrication d'un pigment nacré orangé selon la revendication 3, qui comprend l'addition d'un substrat lamellaire à une suspension aqueuse et le chauffage de ladite suspension sous agitation, l'addition a) d'une solution aqueuse d'un sel ferrique et d'un sel de magnésium et/ou d'un sel de calcium et b) d'une solution aqueuse alcaline à ladite suspension, tout en la maintenant à pH 2 à 5, l'addition d'une solution aqueuse additionnelle alcaline à ladite suspension jusqu'à obtention d'un pH de 8 à 10, la séparation d'un produit par filtration, son lavage, son séchage et sa calcination à une température non inférieure à 500°C.

6. Peinture, encre, matériau plastique ou produit cosmétique contenant un pigment nacré orangé selon l'une quelconque des revendications 1 à 3.
